# EUROPEAN PATENT SPECIFICATION

(11) **EP 4 435 092 B1**
(45) Date of publication and mention of the grant of the patent: **25.02.2026**
(21) Application number: 22910201.7
(22) Date of filing: 23.12.2022
(51) Int. Cl.: C12N 5/00, C12N 5/077, B33Y 70/00, A61L 27/38, A61L 27/50, A61L 27/36

(54) **STROMAL MATERIAL FOR ENCAPSULATING CELLS, PREPARATION METHOD THEREFOR, AND APPLICATION THEREOF**
STROMAMATERIAL ZUR EINKAPSELUNG VON ZELLEN, HERSTELLUNGSVERFAHREN DAFÜR UND ANWENDUNG DAVON
MATÉRIAU STROMAL POUR L'ENCAPSULATION DE CELLULES, SON PROCÉDÉ DE PRÉPARATION ET SON APPLICATION

(30) Priority: 24.12.2021 CN 202111598820
(43) Date of publication of application: 25.09.2024
(73) Proprietor: Beijing Ruijian Gaoke Biotechnology Co., Ltd., Beijing 102200 (CN)
(72) Inventor: JIA, Qipeng, Zhongwei, Ningxia 755006 (CN); SUN, Wenquan, Beijing 102200 (CN)
(74) Representative: Meissner Bolte Nürnberg
(86) International application number: PCT/CN2022/141455
(87) International publication number: WO 2023/116891

(56) References cited:
- WO-A1-2016/024025
- WO-A1-2017/025053
- WO-A1-2020/184974
- WO-A2-2011/132089
- WO-A2-2017/100600
- CN-A- 102 188 748
- CN-A- 104 971 380
- CN-A- 105 288 737
- CN-A- 105 682 697
- CN-A- 106 267 346
- CN-A- 109 621 010
- CN-A- 113 577 391
- CN-A- 114 276 974
- US-A1- 2014 004 549
- US-A1- 2015 126 453
- US-B2- 7 338 757
- EMI A. KIYOTAKE; EMILY C. BECK; MICHAEL S. DETAMORE: "Cartilage extracellular matrix as a biomaterial for cartilage regeneration", ANNALS OF THE NEW YORK ACADEMY OF SCIENCES, NEW YORK ACADEMY OF SCIENCES., US, vol. 1383, no. 1, 9 November 2016 (2016-11-09), US , pages 139 - 159, XP071409889, ISSN: 0077-8923, DOI: 10.1111/nyas.13278
- LIU QIAN, LI XUEJIAN, WANG ZHONGSHAN: "Effects of porcine acellular cartilaginous matrix on the proliferation and differentiation of human adipose-derived stromal cells", WEST CHINA JOURNAL OF STOMATOLOGY., vol. 38, no. 2, 1 April 2020 (2020-04-01), pages 122 - 127, XP093073633, DOI: 10.7518/hxkq.2020.02.002
- ZHANG PEILING, CI ZHENG, JIA LITAO, LIU YU; CAO YILIN, ZHOU GUANGDONG: "Fabrication of Acellular Cartilage Matrix Biomimetic Scaffolds and Their Effects on Chondrogenic Differentiation of Bone Marrow Mesenchymal Stem Cells", ZUZHI GONGCHENG YU CHONGJIAN WAIKE ZAZHI, SHANG HAI JIAO TONG DA XUE YI XUE YUAN FU SHU DI JIU REN MIN YI YUAN, CN, vol. 17, no. 1, 1 January 2021 (2021-01-01), CN , pages 19 - 24, XP093073634, ISSN: 1673-0364, DOI: 10.3969/j.issn.1673-0364.2021.01.003
- JIA LITAO, YAO LIN, LIU YANQUN, ZHANG PEILING, LIU YU, CAO YILIN, ZHOU GUANGDONG: "In Vitro Reconstruction of Tissue Engineering Cartilage Using Acellular Cartilage Matrix Biomimetic Scaffolds", JOURNAL OF TISSUE ENGINEERING AND RECONSTRUCTIVE SURGERY, vol. 15, no. 4, 1 January 2019 (2019-01-01), CN , pages 222 - 244, XP093073635, ISSN: 1673-0364, DOI: 10.3969/j.issn.1673-0364.2019.04.002

## Description

### Technical Field

The present application belongs to a technical field of bioengineering, and particularly to a stromal material for encapsulating cells, a preparation method therefor, and use thereof.

### Background

Cell therapy refers to the treatment method in which normal cells or cells modified by biotechnology are expanded in vitro and then transplanted or infused into patients. Newly infused cells can replace damaged cells to rebuild tissue structure and function (stem cell therapy technology), or have stronger immune killing functions (immune cell therapy technology) to achieve the purpose of treating diseases. Stem cell therapy technology takes advantage of the characteristics of stem cells' self-renewal ability, multi-differentiation potential and high proliferation ability and transplants healthy stem cells into the human body to achieve the purpose of repairing lesions or rebuilding normal tissue functions. Immune cell therapy technology collects immune cells from human body, reinfuses them into the human body after in vitro modification, culture and expansion, and uses the enhanced targeted killing function to kill pathogens, cancer cells, and mutant cells, and activate and enhance immunity ability of the body. With the rapid development of the field such as molecular biology, stem cell biology, tissue engineering and regenerative medicine, the cell therapy has become more and more important in clinical practice. At present, there are many types of cells used in clinical treatment, including bone marrow stem cells, hematopoietic stem cells, neural stem cells, skin stem cells, islet stem cells, adipose stem cells, and various immune cells such as DC, CIK, NK, CD3AK and yδT; and the cells are widely used in the treatment of various clinical diseases, including blood diseases, organ transplantation, cardiovascular system diseases, liver diseases, nervous system diseases, cartilage and bone tissue diseases, tissue trauma and malignant tumors and the like.

Cell culture, proliferation, preservation and transportation are indispensable links to achieve the cell therapy. At present, there are two main ways of cell expansion. One is 2D culture in vitro, which subject cells for subculture and expansion when cells attached proliferates to a confluent state; the shortcoming of the method is that 2D culture in vitro has a low efficiency, and is not conducive to the maintenance of the phenotype of cells. The second is in vitro 3D culture, which immobilizes cells on suitable carriers; and can achieve high-density culture and production of the cells when combined with bioreactors, such as the use of porous microcarrier cell immobilization technology. There are a variety of materials that can be used to prepare porous microcarriers, including polymer synthetic materials with good biocompatibility, purified or structurally modified natural materials (such as starch, cellulose, chitosan, sodium alginate, collagen, gelatin, etc.). Cell clusters culture technology can also be used to achieve carrier-free immobilization of cells, that is, controlling the formation and deaggregation of cell clusters during cell culture to monitor cell growth and achieve efficient cell culture.

Extracellular matrix (ECM) is a biomacromolecular substance that exists between cells and is secreted by cells, which consists mainly of collagen and contains elastin, proteoglycan, glycosaminoglycan and cytokine. Extracellular matrix has a complex spatial network structure, provides a microenvironment for cell growth and activity. The interaction between cells and extracellular matrix has a regulatory effect on cell functions and behaviors such as adhesion, growth, proliferation, differentiation, migration, and intercellular signal transmission. Therefore, extracellular matrix materials can be used to prepare microcarriers for cell immobilization, can be used as active materials to promote a formation of cell clusters, and can be used as cell encapsulation stroma (CES) for encapsulation and protection of cells during cell preservation and transportation process. Patent CN105288737A discloses a tissue engineering cartilage composite scaffold based on cartilage extracellular matrix and a preparation method thereof, which relates to rapidly expanding cartilage seed cells with cartilage extracellular matrix particles and inducing stem cells to differentiate into chondrocytes. The specific preparation method of that invention includes: 1) performing low-temperature wet crushing and sieving of fresh articular cartilage to obtain cartilage particles with a diameter of 100-500 µm, and preparing cartilage extracellular matrix microcarrier after decellularization treatment; 2) placing cartilage extracellular matrix microcarriers and cartilage seed cells into a bioreactor for co-culture, and forming cartilage microtissues by rapid expansion of chondrocytes or induction of stem cells to differentiate into chondrocytes; and 3) filling cartilage microtissue in the pore of the three-dimensional porous scaffold containing hydrogel precursor fluid, and combining the cartilage microtissue with the three-dimensional porous scaffold by coagulant, and obtaining the tissue engineering cartilage composite scaffold by co-culture. There are many studies on cell co-culture and promotion of directed differentiation of stem cells using extracellular matrix microparticles or hydrogels.

WO 2020/184974 A1 discloses a method for producing a composition for cartilage regeneration using freeze-dried cartilage powder the method comprising: A) preparing hyaline cartilage and slicing it; B) freeze-drying and pulverizing the free cartilage to prepare freeze-dried free cartilage powder; C) preparing an adipose tissue extract from autologous adipose tissue; and D) comprising the step of preparing a composition for cartilage regeneration comprising the freeze-dried free cartilage powder and the adipose tissue extract. The composition can be used as bioink.

### Summary

The invention is set out in the appended set of claims. Embodiments of the invention are those whose scope is within that of the appended claims. Any references in the description to methods of treatment refer to the products of the present invention for use in a method for treatment.

Other passages stating embodiments which do not fall under the scope of the appended claims are to be considered as examples.

The following is a summary of subject matters described herein in detail. This summary is not intended to limit the protection scope of claims.

In a first aspect, the present application provides a preparation method for a stromal material for encapsulating cells, the preparation method includes the following steps:
(1) removing tissues and fasciae from a surface of an ex vivo cartilage material of a mammal, and then cutting the cartilage material into thin slices;
(2) disinfecting the surface of a cartilage tissue thin slice with a disinfectant, then washing and freeze-drying;
(3) performing liquid nitrogen low-temperature micronization grinding on the freeze-dried cartilage tissue thin slice to prepare a microparticle material, wherein the average particle size of the microparticle material based on number of particles is 2-20 µm, and the average particle size of the microparticle material based on volume of particles is 20-200 µm;
(4) degreasing the microparticle material by soaking in isopropyl alcohol according to a material-liquid ratio of 1:5 to 1:20, centrifuging, and discarding a waste liquid to obtain a first precipitate;
(5) adding physiological saline or phosphate buffer to the first precipitate according to a material-liquid ratio of 1:5 to 1:20, soaking treatment, centrifuging, and discarding a waste liquid to obtain a second precipitate;
(6) adding a deoxyribonuclease in a buffer solution to the second precipitate according to a material-liquid ratio of 1:5 to 1:20, soaking treatment, centrifuging, and discarding a waste liquid to obtain a third precipitate;
(7) adding an alkaline enzyme (alcalase) solution to the third precipitate according to a material-liquid ratio of 1:5 to 1:10, soaking treatment, centrifuging, and discarding a waste liquid to obtain a fourth precipitate;
(8) adding a detergent which is formulated in a phosphate buffer to the fourth precipitate according to a material-liquid ratio of 1:5 to 1:20, soaking treatment, centrifuging, and discarding a waste liquid to obtain a fifth precipitate;
(9) adding disodium ethylenediamine tetraacetate solution to the fifth precipitate according to a material-liquid ratio of 1:5 to 1:20, soaking, washing, centrifuging, and discarding a waste liquid; adding disodium ethylenediamine tetraacetate solution again for rewashing, recentrifuging, and discarding a waste liquid to obtain a sixth precipitate;
(10) adding physiological saline or phosphate buffer to the sixth precipitate according to a material-liquid ratio of 1:5 to 1:20, soaking treatment, centrifuging, and discarding a waste liquid; adding physiological saline or phosphate buffer again and operating repeatedly to obtain a seventh precipitate;
(11) adding a mixed solution of sodium phosphate and peracetic acid to the seventh precipitate according to a material-liquid ratio of 1:5 to 1:10 to perform virus inactivation treatment, centrifuging, and discarding a waste liquid to obtain a eighth precipitate;
(12) adding physiological saline or phosphate buffer to the eighth precipitate according to a material-liquid ratio of 1:5 to 1:20, soaking treatment, centrifuging, and discarding a waste liquid to obtain a ninth precipitate;
(13) adding sterile water to the ninth precipitate according to a material-liquid ratio of 1:5 to 1:20, washing, freeze-drying, and sterilizing with gamma ray or electron beam after freeze-drying.

In a second aspect, the present application provides a stromal material for encapsulating cells obtained by the preparation method above.

In a third aspect, the present application provides use of the stromal material for encapsulating cells above, that is, the stromal material can be used to promote a formation of cell clusters in a process of cell culture, and can be used to encapsulate and protect cells in the cell preservation and transportation process; or use in 3D bioprinting; or use in the field of medical aesthetics.

### Brief Description of Drawings

FIG. 1 is a scanning electron microscopic image of cartilage matrix microparticles after treatment with alkaline enzyme and decellularization in Example 1 of the present application.
FIG. 2 shows the suspension stability of cartilage matrix microparticles after treatment with alkaline enzyme and decellularization (left) and the suspension stability of cartilage matrix microparticles without treatment with alkaline enzyme but only with decellularization treatment (right) in Example 1 of the present application.
FIG. 3 is a differential scanning calorimetry diagram of untreated pig ear cartilage raw material (top) and cartilage matrix particles after alkaline enzyme treatment and decellularization (bottom) in Example 1 of the present application.
FIG. 4 is a diagram of L929 cells co-cultured on cartilage matrix microparticles after alkaline enzyme treatment and decellularization in Example 1 of the present application.
FIG. 5 is a diagram of cell growth on the cartilage matrix microparticles after 8 hours and 8 days of co-culture of umbilical cord mesenchymal stem cells with cartilage matrix microparticles in Example 1 of the present application.
FIG. 6 is a diagram of the temperature response characteristics of the cartilage matrix microparticle material after alkaline enzyme treatment and decellularization in Example 1 of the present application.
FIG. 7 is a diagram showing the effect of inducing adipose tissue formation by implanting cartilage matrix microparticles into rat subcutaneous tissue after alkaline enzyme treatment and decellularization in Example 1 of the present application.
FIG. 8 is a histological section diagram of pig ear cartilage after alkaline enzyme alcalase treatment for 5 hours (left) and 18 hours (right) in Example 3 of the present application.
FIG. 9 is a general morphology of cartilage matrix material microparticles of different particle sizes in Example 5 of the present application.

### Detailed Description

In an embodiment of a first aspect, the present application provides a preparation method for a stromal material for encapsulating cells, including the steps of:
(1) removing tissues and fasciae from a surface of an ex vivo cartilage material of a mammal, and then cutting the cartilage material into thin slices;
(2) disinfecting the surface of a cartilage tissue thin slicewith a disinfectant, then washing and freeze-drying;
(3) performing liquid nitrogen low-temperature micronization grinding on the freeze-dried cartilage tissue thin slice to prepare a microparticle material, wherein the average particle size of the microparticle material based on number of particles is 2-20 µm, and the average particle size of the microparticle material based on volume of particles is 20-200 µm;
(4) degreasing the microparticle material by soaking in isopropyl alcohol according to a material-liquid ratio of 1:5 to 1:20, centrifuging, and discarding a waste liquid to obtain a first precipitate;
(5) adding physiological saline or phosphate buffer to the first precipitate according to a material-liquid ratio of 1:5 to 1:20, soaking treatment, centrifuging, and discarding a waste liquid to obtain a second precipitate;
(6) adding a deoxyribonuclease in a buffer solution to the second precipitate according to a material-liquid ratio of 1:5 to 1:20, soaking treatment, centrifuging, and discarding a waste liquid to obtain a third precipitate;
(7) adding an alkaline enzyme (alcalase) solution to the third precipitate according to a material-liquid ratio of 1:5 to 1:10, soaking treatment, centrifuging, and discarding a waste liquid to obtain a fourth precipitate;
(8) adding a detergent which is formulated in a phosphate buffer to the fourth precipitate according to a material-liquid ratio of 1:5 to 1:20, soaking treatment, centrifuging, and discarding a waste liquid to obtain a fifth precipitate;
(9) adding disodium ethylenediamine tetraacetate solution to the fifth precipitate according to a material-liquid ratio of 1:5 to 1:20, soaking, washing, centrifuging, and discarding a waste liquid; adding disodium ethylenediamine tetraacetate solution again for rewashing, recentrifuging, and discarding a waste liquid to obtain a sixth precipitate;
(10) adding physiological saline or phosphate buffer to the sixth precipitate according to a material-liquid ratio of 1:5 to 1:20, soaking treatment, centrifuging, and discarding a waste liquid; adding physiological saline or phosphate buffer again and operating repeatedly to obtain a seventh precipitate;
(11) adding a mixed solution of sodium phosphate and peracetic acid to the seventh precipitate according to a material-liquid ratio of 1:5 to 1:10 to perform virus inactivation treatment, centrifuging, and discarding a waste liquid to obtain a eighth precipitate;
(12) adding physiological saline or phosphate buffer to the eighth precipitate according to a material-liquid ratio of 1:5 to 1:20, soaking treatment, centrifuging, and discarding a waste liquid to obtain a ninth precipitate;
(13) adding sterile water to the ninth precipitate according to a material-liquid ratio of 1:5 to 1:20, washing, freeze-drying, and sterilizing with gamma ray or electron beam after freeze-drying.

Preferably, in step (1), the mammal is selected from one or more of pigs, cows, sheep, horses and deer.

Preferably, in step (1), the cartilage material is selected from one or more of elastic cartilage and hyaline cartilage.

Preferably, the elastic cartilage is ear cartilage.

Preferably, the hyaline cartilage is selected from one or more of articular cartilage, costal cartilage, scapular cartilage and meniscus.

Preferably, in step (2), the disinfectant is selected from one or more of 0.1-0.5% w/v sodium hypochlorite, 0.5-2.0% w/v sodium carbonate and 50-70% w/v alcohol solutions.

Preferably, the alcohol solution is selected from one or more of ethanol and isopropanol.

Preferably, in step (4), the isopropanol is at a content of 50-70% w/v; the soaking is for a time of 30-60 min, and the centrifuging is at a speed of 500-1500×g, and for a time of 5-20 min.

Preferably, in step (5), the soaking is for a time of 30-60 min, and the centrifuging is at a speed of 500-1500×g, and for a time of 5-20 min.

Preferably, in step (6), the deoxyribonuclease solution is formulated in a buffer selected from one or more of hydroxyethyl piperazine ethanesulfonic acid buffer and trihydroxymethylaminomethane hydrochloride buffer; the hydroxyethyl piperazine ethanesulfonic acid buffer is at a concentration of 5-100 mmol/L and the deoxyribonuclease solution is at an activity of deoxyribonuclease of 50-250 U/L.

Preferably, in step (6), the soaking is for a time of 8-12 h, and the centrifuging is at a speed of 500-1500×g, and for a time of 5-20 min.

Preferably, in step (7), the alkaline enzyme (alcalase) solution is at a concentration of the alkaline enzyme (alcalase) of 0.02-0.2% w/v.

Preferably, in step (7), the soaking is for a time of 30-120 min, and the centrifuging is at a speed of 500-1500×g, and for a time of 5-20 min.

Preferably, in step (8), the detergent is selected from one or more of 0.5-2% w/v sodium deoxycholate, Triton X-100, and sodium dodecyl sulfate.

Preferably, in step (8), the soaking is for a time of 8-16 h, and the centrifuging is at a speed of 500-1500×g, and for a time of 5-20 min.

Preferably, in step (9), the disodium ethylenediamine tetraacetate solution is at a concentration of disodium ethylenediamine tetraacetate of 5-50 mmol/L, and is formulated in 10 mmol/L sodium phosphate buffer.

Preferably, in step (9), the washing is for a time of 2-4 h, and the centrifuging is at a speed of 500-1500×g, and for a time of 5-20 min.

Preferably, in step (9), the rewashing is for a time of 12-24 h, and the recentrifuging is at a speed of 500-1500×g, and for a time of 5-20 min.

Preferably, in step (10), the soaking is for a time of 30-120 min, and the centrifuging is at a speed of 500-1500×g, and for a time of 5-20 min.

Preferably, in step (11), the virus inactivation treatment is for a time of 30-60 min, and the centrifuging is at a speed of 500-1500×g, and for a time of 5-20 min.

Preferably, in step (12), the soaking is for a time of 30-120 min, and the centrifuging is at a speed of 500-1500×g, and for a time of 5-20 min.

Preferably, in step (5), step (8), step (10) and step (12), the phosphate buffer is a sodium phosphate buffer.

In an embodiment of the present application, the preparation method may be a method for non-disease diagnostic and therapeutic purposes.

In an embodiment of the present application, unless otherwise specified, "% w/v" is "g/100 mL".

In an embodiment of the second aspect, the present application provides a stromal material for encapsulating cells obtained by the preparation method described above.

Preferably, the main components of the stromal material for encapsulating cells are type II collagen and glycosaminoglycan.

Preferably, the main components of the stromal material encapsulating cells are type II collagen, proteoglycan and glycosaminoglycan.

Preferably, the glycosaminoglycan includes chondroitin sulfate and hyaluronic acid.

In some embodiments of the second aspect, the stromal material for encapsulating cells contains type II collagen, chondroitin sulfate and hyaluronic acid.

In some embodiments of the second aspect, the stromal material for encapsulating cells contains type II collagen, chondroitin sulfate and hyaluronic acid, wherein the sum of type II collagen, chondroitin sulfate and hyaluronic acid is above 85% w/w dry weight, preferably above 90% w/w dry weight, more preferably 96.5 ± 0.5% w/w dry weight of the stromal material for encapsulating cells.

In some embodiments of the second aspect, the stromal material for encapsulating cells contains type II collagen above 75% w/w, elastin less than 10% w/w, glycosaminoglycan at a content of 1% to 10% w/w; and the glycosaminoglycan described herein includes chondroitin sulfate and hyaluronic acid.

In some embodiments of the second aspect, the stromal material for encapsulating cells contains type II collagen, chondroitin sulfate and hyaluronic acid, wherein type II collagen, chondroitin sulfate and hyaluronic acid are at a content of 92.4 ± 0.4% w/w dry weight, 2.5 ± 0.09% w/w dry weight and 1.6 ± 0.04% w/w dry weight, or 92.4 ± 0.4% w/w dry weight, 2.5 ± 0.1% w/w dry weight and 1.6 ± 0.0% w/w dry weight, respectively; or above 92% w/w (dry weight), above 2% w/w (dry weight), and above 1% w/w (dry weight).

In some embodiments of the second aspect, residual DNA in the stromal material for encapsulating cells is at a content less than 20 ng/mg, preferably no more than 5 ng/mg, more preferably no more than 2.7 ± 0.6 ng/mg.

In some embodiments of the second aspect, α-Gal antigen epitopes in the stromal material for encapsulating cells is at a number as low as 2.2×10¹² ± 3.0×10¹¹/g dry weight, or less than 5.0×10¹² /g dry weight.

In some embodiments of the second aspect, the stromal material for encapsulating cells contains from 83.5 ± 1.5% w/w to 90.5 ± 1.5% w/w type II collagen.

In a third aspect, the present application provides that the stromal material for encapsulating cells above can be used to promote a formation of cell clusters in a process of cell culture, and can be used to encapsulate and protect cells in the cell preservation and transportation process; provides use of the stromal material for encapsulating cells above in 3D bioprinting; and provides that the stromal material for encapsulating cells above also can be used to fill and induce autologous adipose formation in the field of medical aesthetics.

In some embodiments of the third aspect, the stromal material for encapsulating cells is co-cultured with cells, and does not show obvious cytotoxicity. the stromal material attaches a large number of cells on the surface, showing good performance in supporting the adhesion and proliferation of stem cells. The stromal material can be used as a microcarrier for cell culture, and can also promote the formation of cell clusters.

In some embodiments of the third aspect, the stromal material for encapsulating cells above can encapsulate cells by self-assembly, protect the cells from the influence of a bad environment in the cell preservation and transportation process, and improve the cell viability.

In some embodiments of the third aspect, the stromal material for encapsulating cells above has a protective effect on cells under adverse circumstances.

In some embodiments of the third aspect, the stromal material for encapsulating cells above can be used as a tissue scaffold material, which is injected into the body for tissue regeneration and repair applications; alternatively, it can be used to fill and induce autologous adipose formation in the field of medical aesthetics.

The existing microcarrier material for cell culture is mainly prepared by chemical cross-linking of polymer synthetic materials, purified or structurally modified natural materials (such as starch, cellulose, chitosan, sodium alginate, collagen, gelatin, etc.). However, in the present application, the cartilage of mammals (such as pigs, cattle, sheep, horses, deer, etc.) is used as raw material, and the stromal material for encapsulating cells prepared, the main components of which are natural type II collagen and proteoglycan (chondroitin sulfate and hyaluronic acid), is free of cross-linking agent toxicity, has the advantages of being low immunogenicity, good biocompatibility, and is capable of being implanted into human body.

Structurally, the stromal microparticle material for encapsulating cells prepared based on cartilage extracellular matrix in the present application has a good surface topological structure, and can provide an in vivo-like tissue microenvironment for in-vitro cell culture, thus not only can be used as a microcarrier for cell culture, but also can promote the formation of cell clusters. Functionally, the stromal microparticle material has reversible temperature response characteristics, showing a liquid sol-like state above 35 °C, and a gel state at a lower temperature. When the stromal microparticle material transforms into a gel from a sol, it encapsulates the cells by self-assembly. Using the characteristics of the stromal microparticle material that encapsulates cells by self-assembly when transforming into a gel from a sol, the cells are encapsulated in a gel-state stroma in the cell preservation and transportation process, such that the cells can be protected from the influence of a bad environment, and improving cell viability.

Cartilage matrix microparticles or hydrogels in a current technology are prepared by grinding cartilage materials, acid-base treatment or grinding by conventional pulverizers (such as patents CN106075584A and CN112316211A), and the thermal effect during the grinding process causes proteins in the material being modified or denatured, and reduces the stability of the material. The stromal microparticle material for encapsulating cells prepared based on cartilage extracellular matrix in the present application has a thermal stability above 40 °C, and can be used for 3D bioprinting independently or together with encapsulated cells to form stable tissues and organoid structures.

The method for preparing a stromal microparticle material for encapsulating cells based on cartilage extracellular matrix of the present application employs an alkaline enzyme (alcalase) and has the characteristics of simplicity and high efficiency, which is conducive to large-scale preparation. Cartilage of mammals is extremely dense, and the decellularization process takes a long time, making it difficult to achieve complete and thorough removal of cellular components. Repeating decellularization treatment and prolonged decellularization cycle for multiple times to achieve thorough cell removal, not only takes long time, but also leads to shortcomings of cartilage extracellular matrix being not stable enough, and large loss of components. Treatment with an alkaline enzyme (alcalase) can also reduce α-Gal antigenic epitopes in xenogeneic tissues.

### Specific Implementation Modes

The present application provides a stromal material for encapsulating cells, a preparation method therefor, and use thereof; as well as a stromal material that can be used to promote cell clusters formation in a process of cell culture, cell encapsulation by self-assembly and deaggregation through reversible temperature response characteristics, and can be used to encapsulate and protect cells in the cell preservation and transportation process. The material is a composite natural biomaterial prepared from cartilage of mammals (such as pigs, cattle, sheep, horses, deer, etc.), and the main components are type II collagen and glycosaminoglycan (chondroitin sulfate and hyaluronic acid). The stromal material for encapsulating cells in the present application contains above 75% w/w type II collagen, less than 10% w/w elastin, and a content from 1% to 10% w/w glycosaminoglycan (chondroitin sulfate and hyaluronic acid).

The present application is a method for preparing a stromal material for encapsulating cells based on cartilage materials of animal, which is optionally a method for non-disease diagnostic and therapeutic purposes, including the following steps:
(1) collecting cartilage materials of mammals (such as pigs, cattle, sheep, horses, deer, etc.), including elastic cartilage and hyaline cartilage such as ear cartilage, articular cartilage, costal cartilage, scapular cartilage, meniscus; removing excess tissues and fasciae from the cartilage surface, and cutting the cartilage tissue into thin slices with a thickness less than 2 mm;
(2) disinfecting a surface of a cartilage tissue thin slice with a disinfectant, washing with purified water after disinfection, and freeze-drying until the water content is less than 5% w/w after washing; wherein, the disinfectant is selected from one or more of 0.1-0.5% w/v sodium hypochlorite, 0.5-2.0% w/v sodium carbonate, and 50-70% w/v ethanol (or isopropanol);
(3) performing liquid nitrogen low-temperature micronization grinding on a freeze-dried cartilage tissue thin slice to prepare a microparticle material having an average particle size of 2-20 µm based on number of particles and an average particle size of 20-200 µm based on volume of particles;
(4) degreasing the microparticle material by soaking in 50-70 % w/v isopropyl alcohol according to a material-liquid ratio of 1:5 to 1:20 for 30-60 min, centrifuging at 500-1500×g for 5-20 min and discarding a waste liquid to obtain a first precipitate;
(5) adding 0.9 % w/v physiological saline or 10 mmol/L phosphate buffer (pH=7.4) to the first precipitate according to a material-liquid ratio of 1:5 to 1:20, soaking treatment for 30-60 min, centrifuging at 500-1500×g for 5-20 min and discarding a waste liquid; repeating washing once to obtain a second precipitate;
(6) adding a deoxyribonuclease solution to the second precipitate according to a material-liquid ratio of 1:5 to 1:20, and soaking, wherein the deoxyribonuclease solution is formulated in a buffer selected from one or more of hydroxyethyl piperazine ethanesulfonic acid buffer and trihydroxymethylaminomethane hydrochloride buffer, the hydroxyethyl piperazine ethanesulfonic acid buffer is at a concentration of 5-100 mmol/L, pH=7.6; the deoxyribonuclease solution is at an activity of deoxyribonuclease of 50-250 U/L, after soaking treatment for 8-12 hours, centrifuging at 500-1500×g for 5-20 min, discarding a waste liquid to obtain a third precipitate;
(7) adding an alkaline enzyme (alcalase) solution to the third precipitate according to a material-liquid ratio of 1:5 to 1:10, and soaking, wherein the alkaline enzyme (alcalase) in the solution is at a concentration of 0.02-0.2% w/v, after soaking treatment for 30-120 min, centrifuging at 500-1500×g for 5-20 min, and discarding a waste liquid to obtain a fourth precipitate;
(8) adding a detergent to the fourth precipitate according to a material-liquid ratio of 1:5 to 1:20, wherein the detergent in the solution is selected from one or more of 0.5-2% w/v sodium deoxycholate, Triton X-100 and sodium dodecyl sulfate, and is formulated in 10 mmol/L phosphate buffer, pH = 7.4; after soaking treatment for 8-16h, centrifuging at 500-1500×g for 5-20 min, and discarding a waste liquid to obtain a fifth precipitate;
(9) adding disodium ethylenediamine tetraacetate solution to the fifth precipitate according to a material-liquid ratio of 1:5 to 1:20 and soaking, disodium ethylenediamine tetraacetate is at a concentration of 5-50 mmol/L, which is formulated in 10 mmol/L phosphate buffer, and the solution pH = 7.4; after washing for 2-4 h; centrifuging at 500-1500×g for 5-20 min and discarding a waste liquid; adding disodium ethylenediamine tetraacetate solution again and rewashing for 12-24 h, recentrifuging at 500-1500×g for 5-20 min and discarding a waste liquid to obtain a sixth precipitate;
(10) adding 0.9 % w/v physiological saline or 10 mmol/L phosphate buffer (pH=7.4) to the sixth precipitate according to a material-liquid ratio of 1:5 to 1:20, soaking treatment for 30-120 min, centrifuging at 500-1500×g for 5-20 min and discarding a waste liquid; adding 0.9 % w/v physiological saline or 10 mmol/L phosphate buffer (pH=7.4) again and resoaking for 30-120 min, centrifuging at 500-1500×g for 5-20 min and discarding a waste liquid to obtain a seventh precipitate;
(11) adding a mixed solution of 2-10% w/v sodium phosphate and 0.05-0.5% w/v peracetic acid to the seventh precipitate according to a material-liquid ratio of 1:5 to 1:10, to perform virus inactivation treatment for 30-60 min, centrifuging at 500-1500×g for 5-20 min and discarding a waste liquid to obtain a eighth precipitate;
(12) adding 0.9% w/v physiological saline or 10 mmol/L phosphate buffer (pH=7.4) to the eighth precipitate according to a material-liquid ratio of 1:5 to 1:20, soaking treatment for 30-120 min, centrifuging at 500-1500×g for 5-20 min and discarding a waste liquid to obtain a ninth precipitate;
(13) adding aseptic water to the ninth precipitate according to a material-liquid ratio of 1:5 to 1:20, washing for 30-60 min; adjusting a concentration of a treated sample to 3-6% w/w and then freeze-drying to a water content less than 5% w/w;
(14) after freeze-drying, sterilizing with gamma ray or electron beam, with a dose of 15-35 kGy for sterilization.

Herein, in step (5), step (8), step (10) and step (12), the phosphate buffer is a sodium phosphate buffer.

In the preparation method for a stromal material for encapsulating cells of the present application, alkaline enzyme and decellularization treatment can be carried out first, and then the cartilage tissue can be crushed.

The stromal material for encapsulating cells of the present application is obtained by the preparation method above.

Herein, the main components of the stromal material for encapsulating cells are type II collagen and proteoglycan (chondroitin sulfate and hyaluronic acid).

The stromal material for encapsulating cells of the present application can be applied in 3D bioprinting and also can be used in the field of medical aesthetics to fill and induce autologous adipose formation.

Technical content of the present disclosure will be described further in detail below with reference to Examples. The following Examples are illustrative, not restrictive, and the scope of protection of the present application cannot be limited by the following Examples. The experimental methods used in the following Examples are conventional methods unless otherwise specified. Materials, reagents, etc. used in the following Examples are commercially available unless otherwise specified.

### Example 1

The preparation method for a stromal material for encapsulating cells of the example optionally was a method for non-disease diagnostic and therapeutic purposes, including the following steps:
(1) collecting pig ear cartilage from freshly slaughtered 6-month-old pigs, and removing skin, connective tissue and fascia from a cartilage surface; cutting the cartilage tissue into small pieces with a thickness less than 2 mm, after disinfecting the surface with 0.2% w/v sodium hypochlorite for 10 min, rinsing with purified water and freeze-drying;
(2) placing freeze-dried cartilage into a 200 mL grinding bottle, soaking the sealed container containing the cartilage sample in liquid nitrogen, and quickly crushing the sample by electromagnetic impactor grinding technology with a SPEX 6875 freezing grinder in an environment at -196 °C; after rewarming, screening for cartilage microparticles having small particle size with a 100 µm mesh; detecting by a laser diffraction particle size analyzer, particle sizes based on a particle number distribution being Dx (10) = 1.8 µm, Dx (50) = 2.9 µm, Dx (90) = 6.0 µm, with an average particle size of 2-10 µm; particle sizes based on a particle volume distribution being Dv (10) = 15 µm, Dv (50) = 67 µm, and Dv (90) = 144 µm, with an average particle size of 30-100 µm;
(3) placing the cartilage microparticles into 70% w/v isopropanol solution for soaking and degreasing, with a material-liquid ratio of 1:5 (that is, adding 5 liters of 70% w/v isopropanol solution to 1 liter cartilage microparticles), after degreasing treatment for 1 h, centrifuging at 500×g for 5 minutes and discarding a supernatant waste liquid to obtain a first precipitate; adding 10 mmol/L sodium phosphate buffer (pH=7.4) according to a material-liquid ratio of 1:5, after soaking treatment for 30 min, centrifuging at 500×g for 5 min and discarding a supernatant waste liquid to obtain a second precipitate; and repeating the washing with sodium phosphate buffer once;
(4) placing the degreased cartilage microparticles into a deoxyribonuclease solution, with a concentration of hydroxyethyl piperazine ethanesulfonic acid buffer of 10 mmol/L and an activity of deoxyribonuclease at 50 U/L in the buffer for deoxyribonuclease solution, pH = 7.6, and a material-liquid ratio of 1:5; after soaking treatment for 8 h, centrifuging at 500×g for 5 min and discarding a supernatant waste liquid to obtain a third precipitate; adding an alkaline enzyme (alcalase) solution to the third precipitate according to a material-liquid ratio of 1:5, with a concentration of the alkaline enzyme (alcalase) of 0.05% w/v in the solution, which is formulated in 10 mmol/L sodium phosphate buffer, pH = 7.4, after alkaline enzyme (alcalase) treatment for 120 min, centrifuging at 500×g for 5 min and discarding a supernatant waste liquid to obtain a fourth precipitate; after alkaline enzyme (alcalase) treatment, adding sodium deoxycholate solution to the fourth precipitate according to a material-liquid ratio of 1:5, with a concentration of sodium deoxycholate of 1.0% w/v, which is formulated in 10 mmol/L sodium phosphate buffer, pH = 7.4, after treatment for 12h, centrifuging at 500×g for 5 min and discarding a supernatant waste liquid to obtain a fifth precipitate;
(5) adding the degreased, enzyme treated and detergent washed materials into EDTA solution according to a material-liquid ratio of 1:5, with a concentration of EDTA of 10 mmol/L, which is formulated in 10 mmol/L sodium phosphate buffer, pH = 7.4, soaking and washing for 2 h, centrifuging at 500×g for 5 min and discarding a supernatant waste liquid to obtain a sixth precipitate; and washing with EDTA solution once again; and then using 10 mmol/L sodium phosphate buffer (pH = 7.4) to soak and wash for 2 h, centrifuging at 500×g for 5 min and discarding a supernatant waste liquid to obtain a seventh precipitate; and washing with sodium phosphate solution once again;
(6) adding the washed material to a mixed solution containing 2% w/v sodium phosphate and 0.2% w/v peracetic acid for virus inactivation treatment according to a material-liquid ratio of 1:5, after inactivation treatment for 60 min, centrifuging at 500×g for 5 min and discarding a supernatant waste liquid to obtain a eighth precipitate; adding 0.9% w/v physiological saline according to a material-liquid ratio of 1:5, soaking treatment for 60 min, centrifuging at 500×g for 5 min and discarding a supernatant waste liquid to obtain a ninth precipitate;
(7) washing the ninth precipitate twice with sterile purified water: adding sterile water into the ninth precipitate according to a material-liquid ratio of 1:5 for washing, 30 min each time, then centrifuging at 500×g for 5 min and discarding a supernatant waste liquid; adjusting a dry weight concentration of treated microparticle samples to 3% w/v and then freeze-drying; after freeze-drying, sterilizing with gamma ray, with irradiation dose of 17.1 kGy; preparing and obtaining a stromal material for encapsulating cells based on cartilage material.

A content of residual DNA in cartilage matrix microparticles prepared by the method above was 2.7 ± 0.6 ng/mg dry weight; which was decreased by 99.6%, compared with untreated freeze-dried cartilage raw material (679.8 ± 7.3 ng/mg by dry weight). A collagen content in the treated cartilage matrix microparticles was 92.4 ± 0.4% w/w by dry weight, and that in the untreated freeze-dried cartilage materials was 50.9 ± 5.2% w/w by dry weight, indicating that alkaline enzyme alcalase treatment and decellularization treatment effectively removed other tissue proteins. A content of chondroitin sulfate in treated cartilage matrix microparticles was 25.5 ± 0.9 ug/mg dry weight (2.55 ± 0.09% w/w by dry weight), and that in untreated freeze-dried cartilage materials was 80.5 ± 4.1 ug/mg dry weight (8.05 ± 0.41% w/w by dry weight). A content of hyaluronic acid in the treated cartilage matrix microparticles was 16.6 ± 0.4 ug/mg dry weight (1.66 ± 0.04% w/w by dry weight), and that in the untreated freeze-dried cartilage raw materials was 136.6 ± 7.3 ug/mg by dry weight (13.66 ± 0.73% w/w by dry weight). The results of component analysis showed that the treated cartilage matrix microparticles were natural composite materials of collagen, chondroitin sulfate and hyaluronic acid, and a sum of the above three was 96.5 ± 0.53% w/w by dry weight.

The cartilage matrix microparticles prepared by the method above were rehydrated with a 0.9% w/v sodium chloride solution, and then detected by a laser diffraction particle size analyzer. A particle size of the prepared cartilage matrix materials in terms of number distribution were Dx (10) = 2.8 µm, Dx (50) = 4.0 µm, Dx (90) = 8.7 µm, and a number average particle size of the particles was 3-8 µm; and a particle size based on the particle volume distribution were Dv (10) = 13 µm, Dv (50) = 80 µm, and Dv (90) = 400 µm, and a volume average particle size of the particle was 40-200 µm. The particle size of cartilage matrix particle material was increased after alkaline enzyme treatment and decellularization treatment.

A number of α-Gal antigen epitopes was quantitatively detected for cartilage matrix microparticle material prepared by the method above with reference to the standard "Tissue engineering medical device products - Remnant α-Gal antigen determination in scaffold materials utilizing animal tissues and their derivatives" (YY/T 1561-2017). The number of α-Gal antigen epitopes in untreated pig ear cartilage was 5.4×10¹³± 5.2×10¹² /g dry weight material, the number of α-Gal antigen epitopes in treated cartilage matrix particle material as low as 2.2×10¹²± 3.0×10¹¹ /g dry weight material, and alkaline enzyme treatment and decellularization removed 95.9% of α-Gal antigen epitopes.

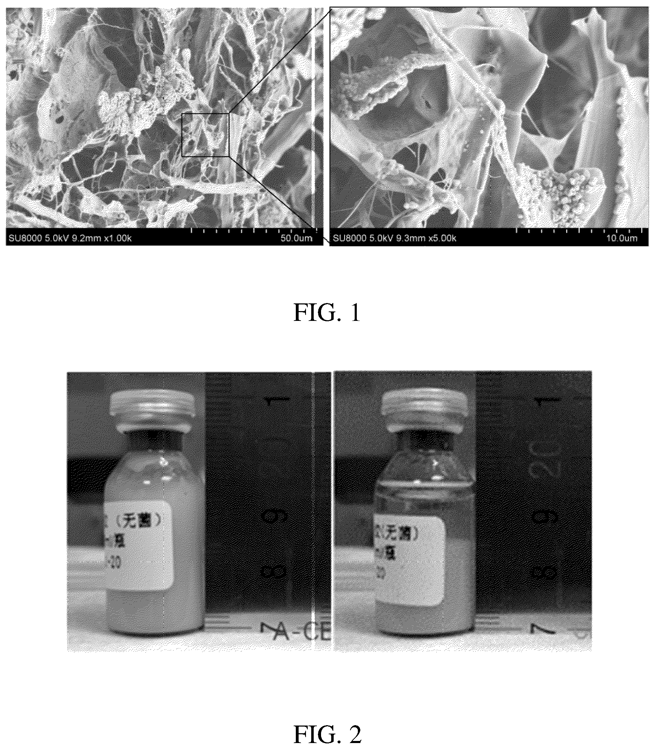

A surface topology of cartilage matrix particle has important effects on response acitivities such as cell adhesion and proliferation, and has important physiological effects on cell morphology, phenotype and cell movement. A surface structure of the cartilage matrix microparticles prepared by the alkaline enzyme treatment and the decellularization process in this example was observed by an ultra-high resolution field emission scanning electron microscope (SU8000), and the results showed that the cartilage matrix microparticles had a three-dimensional porous uneven rough surface structure with a large surface area (FIG. 1).

The cartilage matrix microparticle material prepared by the method above was formulated into a 4% w/v suspension with 0.9% w/v sodium chloride solution (physiological saline), and after standing for 5 days, no sedimentation or separation occurs, showing that the cartilage matrix microparticle material had good suspension stability (FIG. 2). If there was only decellularization treatment but without alkaline enzyme alcalase treatment, the microparticle material would appear sedimentation, indicating its poor suspension stability. An improvement in the suspension stability of the cartilage matrix microparticles after alkaline enzyme treatment and decellularization was related to an increase in the particle size of cartilage matrix particles after treatment, which reduced the density of cartilage matrix particles.

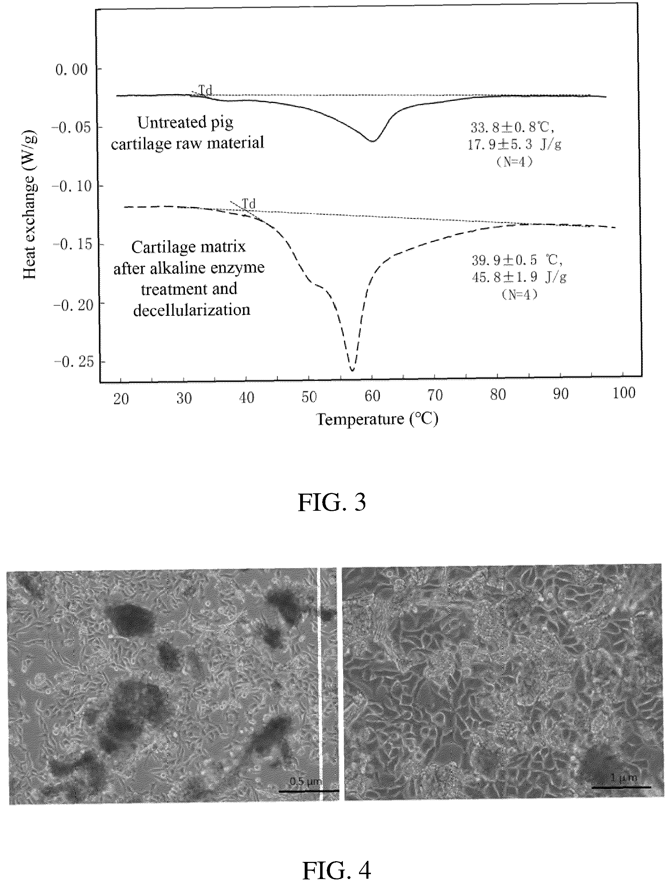

A thermal stability of the cartilage material rehydrated in physiological saline and the prepared cartilage matrix particle material was compared using a differential scanning calorimeter (DSC) (FIG. 3). The thermal stability of the cartilage matrix particle prepared by the enzyme treatment and decellularization method of the present application was good, and above 40°C, basically similar to that of the untreated cartilage material, indicating that there was no damage during the preparation process. Because of the increase of collagen content, an enthalpy of the prepared cartilage matrix microparticles during protein denaturation was significantly increased. In addition, the cartilage matrix microparticles were placed at 37 °C for 24 h and then tested by DSC, and the DSC thermogram did not change, and the results also showed the thermal stability of cartilage matrix particles.

In order to test whether the cartilage matrix microparticles had good biosafety after alkaline enzyme treatment and decellularization, L929 cells were co-cultured with cartilage matrix microparticles for 48 hours. The results showed that the cells appeared a relatively obvious aggregation growth phenomenon, indicating that the material had good safety, which indicates to a certain extent that the material has good biocompatibility (Fig. 4).

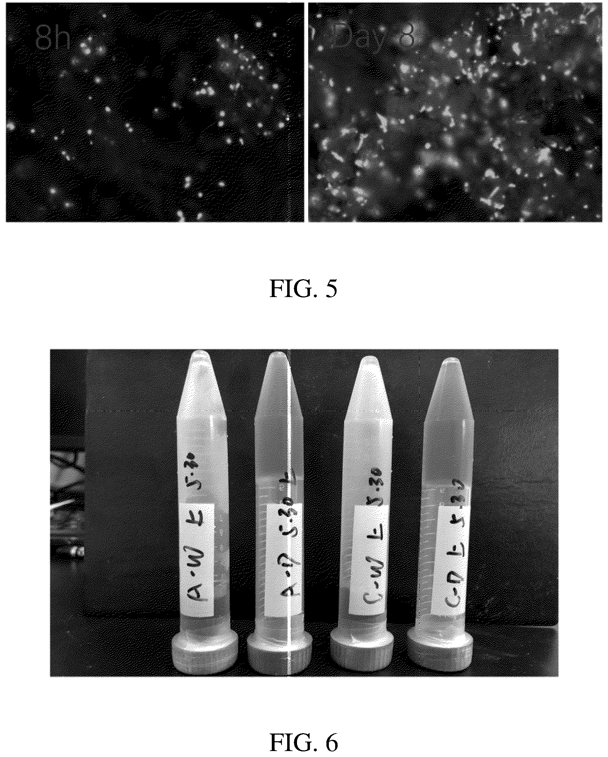

In order to further test the ability of cartilage matrix microparticles after alkaline enzyme treatment and decellularization to support cell adhesion and proliferation, cartilage matrix microparticles were co-cultured with human umbilical cord mesenchymal stem cells to observe their cell adhesion and cytotoxic effects. The experimental results showed that the material did not show obvious cytotoxicity in co-culture, and cartilage matrix microparticles have a large number of cells to their surface, showing good performance in supporting stem cell adherence and proliferation (Fig. 5). Cartilage matrix-based materials could be used as microcarriers for cell culture, and could also promote cell clusters formation.

The cartilage matrix microparticle material prepared by the method above had a reversible temperature response characteristic in function (FIG. 6). The cartilage matrix microparticle material presented a liquid sol-like state and had fluidity at 37 °C; and presented a gel state and did not have fluidity when the temperature dropped. When transforming into a gel from a sol, the cartilage matrix microparticle material can encapsulate cells by self-assembly. Taking advantage of characteristics that the cartilage matrix microparticle can encapsulate cells by self-assembly when transforming into a gel from a sol, it can protect the cells from the influence of a bad environment and improve the cell viability in the cell preservation and transportation process, Because the temperature response characteristics of cartilage matrix microparticles were reversible, the cells can be depolymerized when the temperature rised to 37 °C.

L929 cells were co-cultured with 2.5% cartilage matrix microparticles at 37 °C for 48 hours, and the cell suspension was transferred to a constant temperature metal bath at 25 °C and a refrigerator at 4 °C for storage, which was used to test the protective effect of cartilage matrigel on cells in adversity. The activity of L929 cells was detected by CCK-8 kit on day 1 and day 4 after being transferred to low temperature environment. The activity of cell was 0.424 ± 0.008 and 0.459 ± 0.022 on day 1 and day 4 in a constant temperature metal bath at 25 °C, respectively, and there was no change in cell activity; and the activity of cell was 0.410 ± 0.012 and 0.344 ± 0.020 on day 1 and day 4 in a 4 ° C refrigerator, respectively, with a slight decrease in cell activity.

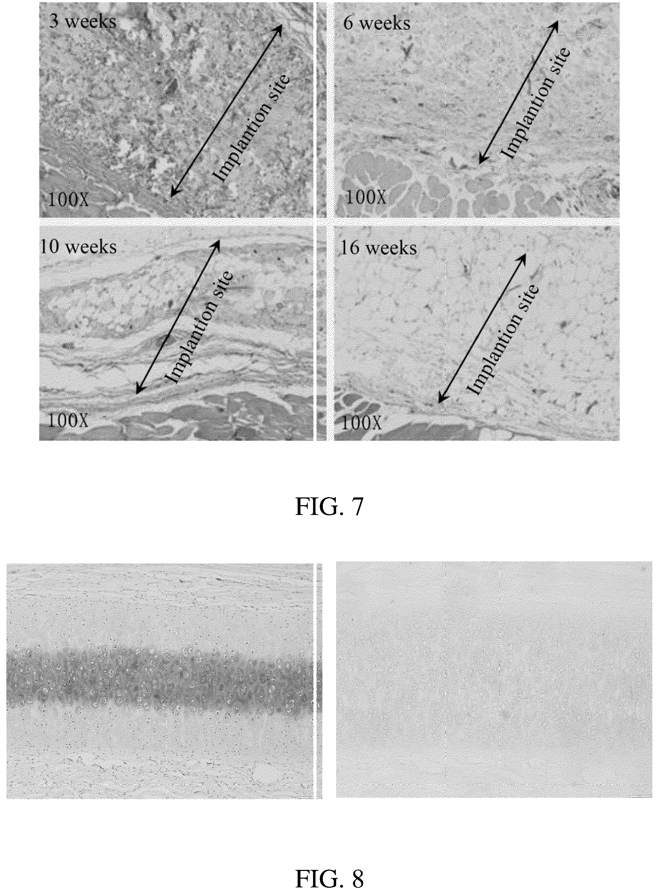

The cartilage matrix microparticle material prepared by the method above was implanted subcutaneously into the back of rats by injection, which could play a good role as a scaffold and could induce a large amount of adipose tissue formation (Fig. 7). 3 weeks after implantation, an inflammatory response can be seen, but the degree of inflammation decreased with the prolongation of time, and there was no chronic inflammatory response; at 6 weeks, neoangiogenesis occurred in the cartilage matrix microparticles region, and the cartilage matrix microparticles degraded to a certain extent over time; at 10 weeks, adipocytes emerged and entered into growth, and at 16 weeks, the region were completely filled with adipocytes. Therefore, the cartilage matrix microparticles could also be used in the field of medical aesthetics to fill and induce autologous adipose formation.

### Example 2

A preparation method for cartilage matrix material of the present example was a method for non-disease diagnostic and therapeutic purposes, the steps of which were identical to Example 1 except for the following step. In step (4), after soaking in the deoxyribonuclease solution, the sodium deoxycholate solution was directly added for treatment without the alkaline enzyme (alcalase) solution treatment.

The cartilage matrix microparticles prepared by the method of Example 2 were substantially different from the cartilage matrix microparticles prepared by the method of Example 1 (table 1). After treatment by the method of Example 1, the residual DNA content of the cartilage matrix microparticles was lower, and the number of the residual α-Gal antigen epitopes was less; after treatment by the method of Example 2, the residual DNA content of the cartilage matrix particles was higher, and the number of the residual α-Gal antigen epitopes was more. accumulated contents of collagen, chondroitin sulfate and hyaluronic acid in the cartilage matrix microparticle material treated by the method of Example 1 were 96.5 ± 0.53% w/w dry weight; however, the accumulated contents of collagen, chondroitin sulfate and hyaluronic acid in the cartilage matrix microparticle material treated by the method of Example 2 were only 75.1 ± 5.2% w/w dry weight. The test results showed that the effect of alkaline enzyme (alcalase) treatment was significant.

**Table 1. Comparison of Method of Example 1 and Method of Example 2**

| Detection index | Method of Example 1 | Method of Example 2 |
|---|---|---|
| Residual DNA content (ng/mg); | 2.7 ± 0.6 | 7.2 ± 2.7 |
| Collagen content (% w/w) | 92.4 ± 0.4 | 61.9 ± 5.2 |
| Chondroitin sulfate content (% w/w) | 2.5 ± 0.09 | 7.1 ± 0.7 |
| Hyaluronic acid (% w/w) | 1.6 ± 0.04 | 6.1 ± 1.0 |
| Number of α-Gal antigen epitopes (/g dry weight) | 2.2×10¹²± 3.0×10¹¹ | 7.4×10¹²± 1.1×10¹² |

### Example 3

The preparation method for a stromal material for encapsulating cells of the present example was method for non-disease diagnostic and therapeutic purposes, including the following steps:
(1) collecting pig ear cartilage from freshly slaughtered 6-month-old pigs, and removing other tissues, and cutting the cartilage with a thickness of about 2 mm into small pieces of 2 cm×3 cm, adding 0.5% w/v sodium hypochlorite solution according to a material-liquid ratio of 1:5, to perform disinfecting treatment for 10 min, then discarding a waste liquid; adding 70% w/v isopropanol to degrease for 60 min, then discarding a waste liquid and soaking in 0.125% w/v alkaline enzyme (alcalase) solution for 18 h;
(2) adding the mixed solution containing 2% w/v sodium phosphate and 0.15% w/v peracetic acid according to a material-liquid ratio of 1:5 to the alkaline enzyme treated material for virus inactivation treatment, after the virus inactivation treatment for 120 min, discarding a waste liquid; then adding an EDTA solution, with a concentration of EDTA of 10 mmol/L, which is formulated with 10 mmol/L sodium phosphate buffer, pH = 7.4, according to a material-liquid ratio of 1:5, soaking and washing for 24 h, then discarding a waste liquid, and washing with EDTA solution for 24 h repeatedly;
(3) washing with physiological saline for 24 h.

In the process of preparing cartilage decellularized matrix material according to method of Example 3, after alkaline enzyme (alcalase) treatment for 5 h, taking samples to detect the decellularizing progress. After alkaline enzyme (alcalase) treatment for 5 h, there was a cell-containing area similar to that of untreated cartilage in the middle of cartilage tissue, but after alkaline enzyme treatment for 18 h, the whole cartilage had been completely changed; a content of the residual DNA was 6.79 ± 0.65ng/mg dry weight. Tissue sections of pig ear cartilage treated with the alkaline enzyme alcalase for 5 h (left) and 18 h (right) were shown in Fig. 8. After alkaline enzyme treatment, the collagen content of cartilage matrix was 76.5%±6.5% w/w dry weight, indicating that alkaline enzyme (alcalase) treatment could also effectively effect cartilage decellularization; however, the use of intact cartilage was significantly less efficient compared to the micronizated material (Example 1).

### Example 4

The preparation method for a stromal material for encapsulating cells of the present example was a method for non-disease diagnostic and therapeutic purposes, including the following steps:
(1) collecting costal cartilage from freshly slaughtered 22-month-old cows, and removing other tissues, cutting cartilage tissue into thin slices with a thickness less than 2 mm; adding 50% w/v isopropanol according to a material-liquid ratio of 1:5 to thin slices of cartilage tissue for degreasing and disinfecting, soaking for 30 min, then centrifuging at 1500×g for 20 min, and discarding a waste liquid to obtain a first precipitate;
(2) to the cartilage tissue slices treated with isopropanol, adding the alkaline enzyme alcalase solution according to a material-liquid ratio of 1:10, which has a 0.2% w/v concentration of the alkaline enzyme and is formulated in 10 mmol/L sodium phosphate buffer, pH = 7.4; after treatment for 30min, centrifuging at 1500×g for 20 min, and discarding a waste liquid to obtain a second precipitate;
(3) to the cartilage tissue slices treated with the alkaline enzyme alcalase, adding Triton X-100 according to a material-liquid ratio of 1:20,which is formulated in 10 mmol/L sodium phosphate buffer, pH = 7.4; after treatment for 16 h, centrifuging at 1500×g for 20 min, and discarding a waste liquid to obtain a third precipitate;
(4) placing the cartilage tissue slices soaked in detergent into 0.9% w/v physiological saline according to a material-liquid ratio of 1:5 for soaking treatment for 30 min, centrifuging at 1500×g for 20 min, and discarding a waste liquid; adding 0.9% w/v physiological saline again and resoaking for 30 min, centrifuging at 1500×g for 20 min, and discarding a waste liquid to obtain a fourth precipitate;
(5) placing the washed cartilage tissue slices in a deoxyribonuclease solution according to a material-liquid ratio of 1:20 for soaking, which contains 5 mmol/L hydroxyethyl piperazine ethanesulfonic acid buffer, and an activity of deoxyribonuclease of 250 U/L, pH = 7.6; after treatment for 12 h, centrifuging at 1500×g for 20 min, and discarding a waste liquid to obtain a fifth precipitate;
(6) to the deoxyribonuclease treated cartilage slices, adding 10 mmol/L sodium phosphate buffer (pH = 7.4) according to a material-liquid ratio of 1:20 for a soaking treatment for 120 min, centrifuging at 1500×g for 20 min, and discarding a waste liquid; adding 10 mmol/L sodium phosphate buffer (pH = 7.4) again and resoaking for 120 min, centrifuging at 1500×g for 5 min, and discarding a waste liquid to obtain a sixth precipitate;
(7) adding a mixture of 10% w/v sodium chloride and 0.5% w/v peracetic acid according to a material-liquid ratio of 1:10 for virus inactivation treatment for 30 minutes, centrifuging at 1500×g for 20 min, and discarding a waste liquid to obtain a seventh precipitate;
(8) placing the virus-inactivated cartilage tissue slices into EDTA solution according to a material-liquid ratio of 1:20 for soaking, which contains a concentration of EDTA of 50 mmol/L, and a solution pH = 7.4; after washing for 4 h, centrifuging at 1500×g for 20 min, and discarding a waste liquid, and washing with EDTA solution once again, centrifuging at 1500×g for 20 min, and discarding a waste liquid to obtain a eighth precipitate;
(9) placing the treated cartilage tissue slices into aseptic water according to a material-liquid ratio of 1:20, washing for 60 min, freeze-drying the washed cartilage tissue slices to a water content less than 5% w/w;
(10) placing freeze-dried cartilage tissue slices into a 200 mL grinding bottle, soaking a sealed container containing the cartilage sample in liquid nitrogen, and quickly crushing the sample by electromagnetic impactor grinding technology with a SPEX 6875 freezing grinder in an environment at -196 °C; after rewarming, screening for cartilage microparticle having small particle size with a 100 µm mesh;
(11) sterilizing the microparticle materials with gamma ray or electron beam to prepare stromal materials for encapsulating cells, with sterilization dose of 15kGy.

The cartilage matrix microparticle material prepared by the method of Example 4 above had similar characteristics to the cartilage matrix microparticle material prepared by the method of Example 1.

### Example 5:

The preparation method for a stromal material for encapsulating cells of the Example includes the following steps of:
(1) collecting pig ear cartilage from freshly slaughtered 6-month-old pigs, and removing other tissues, adding 70% w/v isopropanol to degrease for 60 min, then discarding a waste liquid; washing with purified water and freeze-drying;
(2) placing freeze-dried cartilage into a sealed container and soaking in liquid nitrogen, and frozen grinding in an environment of -196°C (see Example 1); after low-temperature grinding, using sieve to screen according to particle size to obtain cartilage microparticles of different sizes ( < 0.1 mm, 0.1-0.2 mm, 0.2-0.3 mm, 0.3-0.4 mm);
(3) soaking cartilage microparticles of different particle sizes in a deoxyribonuclease solution, with 12 mL of solution being added per gram of dry weight of cartilage microparticles; the solution containing 5 mmol/L hydroxyethyl piperazine ethanesulfonic acid buffer, with the activity of deoxyribonuclease in deoxyribonuclease solution being 500 U/L (pH = 7.6), after treatment at 37°C for 16 h, centrifuging at 1500×g for 20 min, and discarding a waste liquid;
(4) adding 6 mL of 0.05% w/v the alkaline enzyme Alcalase per gram of cartilage microparticles dry weight for a treatment at 37 °C for 2 h, then centrifuging at 1500×g for 20 min, and discarding a waste liquid;
(5) adding 6mL 0.5% w/v sodium deoxycholate (SDOC) per gram of cartilage microparticles dry weight for a treatment at room temperature for 6 h, centrifuging at 1500×g for 20 min, and discarding a waste liquid;
(6) washing at room temperature with phosphate buffer containing penicillin/streptomycin (pH = 7.0, containing 100 U penicillin/streptomycin/mL) for 4 times, each time for 8 h; centrifuging at 1500×g for 20 min to collect precipitated cartilage matrix microparticles;
(7) diluting with 0.9% w/v sodium chloride solution to make the concentration of cartilage matrix microparticles to be 5% w/w;
(8) disinfecting with gamma ray terminal (19.03 kGy-20.08 kGy), sampling and analying.

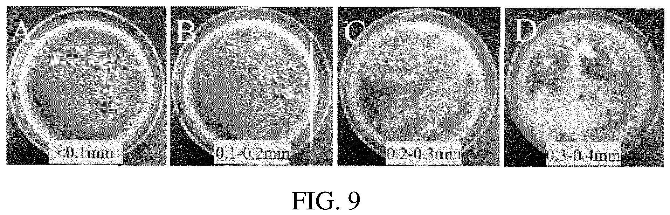

The cartilage stromal materials prepared with cartilage microparticles of different size appeared aggregation phenomenon to a different extent; the extent of aggregation of matrix material increased with the increase in particle size of cartilage microparticles (Fig. 9). Only when the particle size was < 0.1 mm, there was a good dispersion, and when the particle size was > 0.3 mm, obvious aggregation and interweaving phenomena appeared.

The DNA content of cartilage microparticles before decellularization was about 411 ng/mg, and the DNA content after decellularization was less than 20 ng/mg; the content of collagen in cartilage matrix ranged from 83.5 ± 1.2% w/w to 90.5 ± 1.5% w/w, the larger the particle size, the higher the content of collagen; and the content of elastin in cartilage matrix was 1.6-4.0% w/w. The chondroitin sulfate content in cartilage decellularized matrix microcarriers was determined using the Sulfated Glycosaminoglycan (sGAG) assay kit, with a chondroitin sulfate content of 7.1 ± 0.7% w/w.

The cartilage matrix microparticle material prepared by the method of Example 5 above had similar characteristics to the cartilage matrix microparticle material prepared by the method of Example 1.

## Claims

1. A preparation method for a stromal material for encapsulating cells, comprising the following steps of:
(1) removing tissues and fasciae from a surface of an ex vivo cartilage material of a mammal, and then cutting the cartilage material into thin slices to obtain a cartilage tissue thin slice;
(2) disinfecting the surface of the cartilage tissue thin slice with a disinfectant, then washing and freeze-drying;
(3) performing liquid nitrogen low-temperature micronization grinding on the freeze-dried cartilage tissue thin slice to prepare a microparticle material, wherein the average particle size of the microparticle material based on number of particles is 2-20 µm, and the average particle size of the microparticle material based on volume of particles is 20-200 µm;
(4) degreasing the microparticle material by soaking in isopropyl alcohol according to a material-liquid ratio of 1:5 to 1:20, centrifuging, and discarding a waste liquid to obtain a first precipitate;
(5) adding physiological saline or phosphate buffer to the first precipitate according to a material-liquid ratio of 1:5 to 1:20, soaking treatment, centrifuging, and discarding a waste liquid to obtain a second precipitate;
(6) adding a deoxyribonuclease in a buffer solution to the second precipitate according to a material-liquid ratio of 1:5 to 1:20, soaking treatment, centrifuging, and discarding a waste liquid to obtain a third precipitate;
(7) adding an alkaline enzyme (alcalase) solution to the third precipitate according to a material-liquid ratio of 1:5 to 1:10, soaking treatment, centrifuging, and discarding a waste liquid to obtain a fourth precipitate;
(8) adding a detergent which is formulated in a phosphate buffer to the fourth precipitate according to a material-liquid ratio of 1:5 to 1:20, soaking treatment, centrifuging, and discarding a waste liquid to obtain a fifth precipitate;
(9) adding disodium ethylenediamine tetraacetate solution to the fifth precipitate according to a material-liquid ratio of 1:5 to 1:20, soaking, washing, centrifuging, and discarding a waste liquid; adding disodium ethylenediamine tetraacetate solution again for rewashing, recentrifuging, and discarding a waste liquid to obtain a sixth precipitate;
(10) adding physiological saline or phosphate buffer to the sixth precipitate according to a material-liquid ratio of 1:5 to 1:20, soaking treatment, centrifuging, and discarding a waste liquid; adding physiological saline or phosphate buffer again and operating repeatedly to obtain a seventh precipitate;
(11) adding a mixed solution of sodium phosphate and peracetic acid to the seventh precipitate according to a material-liquid ratio of 1:5 to 1:10 to perform virus inactivation treatment, centrifuging, and discarding a waste liquid to obtain a eighth precipitate;
(12) adding physiological saline or phosphate buffer to the eighth precipitate according to a material-liquid ratio of 1:5 to 1:20, soaking treatment, centrifuging, and discarding a waste liquid to obtain a ninth precipitate; and
(13) adding sterile water to the ninth precipitate according to a material-liquid ratio of 1:5 to 1:20, washing, freeze-drying, and sterilizing with gamma ray or electron beam after freeze-drying.

2. The preparation method for a stromal material for encapsulating cells according to claim 1, wherein: in step (1), the mammal is selected from one or more of pigs, cows, sheep, horses and deer;
in step (1), the cartilage material is selected from one or more of elastic cartilage and hyaline cartilage;
the elastic cartilage is ear cartilage;
the hyaline cartilage is selected from one or more of articular cartilage, costal cartilage, scapular cartilage and meniscus.

3. The preparation method for a stromal material for encapsulating cells according to claim 1, wherein: in step (2), the disinfectant is selected from one or more of 0.1-0.5% w/v sodium hypochlorite, 0.5-2.0% w/v sodium carbonate and 50-70% w/v alcohol solution;
the alcohol solution is selected from one or more of ethanol and isopropanol.

4. The preparation method for a stromal material for encapsulating cells according to claim 1, wherein: in step (4), the isopropanol is at a content of 50-70% w/v; the soaking is for a time of 30-60min, and the centrifuging is at a speed of 500-1500×g, and for a time of 5-20 min;
in step (5), the soaking is for a time of 30-60 min, and the centrifuging is at a speed of 500-1500×g, and for a time of 5-20 min;
in step (6), the deoxyribonuclease solution is formulated in a buffer selected from one or more of hydroxyethyl piperazine ethanesulfonic acid buffer and trihydroxymethylaminomethane hydrochloride buffer; the deoxyribonuclease solution is at an activity of deoxyribonuclease of 50-250 U/L;
the hydroxyethyl piperazine ethanesulfonic acid buffer is at a concentration of 5-100 mmol/L;
in step (6), the soaking is for a time of 8-12 h, and the centrifuging is at a speed of 500-1500×g, and for a time of 5-20 min.

5. The preparation method for a stromal material for encapsulating cells according to claim 1, wherein: in step (7), the alkaline enzyme (alcalase) solution is at a concentration of alkaline enzyme (alcalase) of 0.02-0.2% w/v;
in step (7), the soaking is for a time of 30-120 min, and the centrifuging is at a speed of 500-1500×g, and for a time of 5-20 min;
in step (8), the detergent is selected from one or more of sodium deoxycholate, Triton X-100, and sodium dodecyl sulfate;
in step (8), the soaking is for a time of 8-16 h, and the centrifuging is at a speed of 500-1500×g, and for a time of 5-20 min.

6. The preparation method for a stromal material for encapsulating cells according to claim 1, wherein: in step (9), the disodium ethylenediamine tetraacetate solution is at a concentration of disodium ethylenediamine tetraacetate of 5-50 mmol/L and is formulated in 10 mmol/L sodium phosphate buffer;
in step (9), the washing is for a time of 2-4 h, and the centrifuging is at a speed of 500-1500×g, and for a time of 5-20 min;
in step (9), the rewashing is for a time of 12-24 h, and the recentrifuging is at a speed of 500-1500×g, and for a time of 5-20 min;
in step (10), the soaking is for a time of 30-120 min, and the centrifuging is at a speed of 500-1500×g, and for a time of 5-20 min.

7. The preparation method for a stromal material for encapsulating cells according to claim 1, wherein: in step (11), the virus inactivation treatment is for a time of 30-60 min, and the centrifuging is at a speed of 500-1500×g, and for a time of 5-20 min;
in step (12), the soaking is for a time of 30-120 min, and the centrifuging is at a speed of 500-1500×g, and for a time of 5-20 min.

8. The preparation method for a stromal material for encapsulating cells according to any one of claims 1-7, wherein: in step (5), step (8), step (10) and step (12), the phosphate buffer is a sodium phosphate buffer.

9. A stromal material for encapsulating cells obtained by the preparation method according to any one of claims 1-8;
main components of the stromal material for encapsulating cells are type II collagen and glycosaminoglycan;
the glycosaminoglycan comprises chondroitin sulfate and hyaluronic acid.

10. The stromal material for encapsulating cells according to claim 9, comprising type II collagen, chondroitin sulfate and hyaluronic acid, wherein a sum of type II collagen, chondroitin sulfate and hyaluronic acid is above 85% w/w dry weight, preferably above 90% w/w dry weight of the stromal material for encapsulating cells;
the type II collagen is above 75% w/w, elastin is less than 10% w/w, and the glycosaminoglycan is at a content from 1% to 10% w/w; and the glycosaminoglycan described herein comprises chondroitin sulfate and hyaluronic acid;
the collagen II, the chondroitin sulfate and the hyaluronic acid are at a content above 92% w/w (dry weight), above 2% w/w (dry weight) and above 1% w/w (dry weight), respectively;
residual DNA in the stromal material for encapsulating cells is at a content less than 20 ng/mg, preferably no more than 5 ng/mg;
α-Gal antigen epitopes in the stromal material for encapsulating cells is at a number below 5.0×10¹² /g dry weight.

11. Use of the stromal material for encapsulating cells of claim 9 or 10 in 3D bioprinting.

12. Use of the stromal material for encapsulating cells of claim 9 or 10 in encapsulating, or protecting, or encapsulating and protecting cells.

## Patentansprüche

1. Verfahren zur Herstellung eines Stromamaterials zum Einkapseln von Zellen, umfassend die folgenden Schritte:
(1) Entfernen von Geweben und Faszien von einer Oberfläche eines ex vivo gewonnenen Knorpelmaterials eines Säugetiers und anschließendes Schneiden des Knorpelmaterials in dünne Scheiben, um eine dünne Knorpelgewebescheibe zu erhalten;
(2) Desinfizieren der Oberfläche der dünnen Knorpelgewebescheibe mit einem Desinfektionsmittel, anschließendes Waschen und Gefriertrocknen;
(3) Durchführen einer Mikronisierung durch Mahlen mit flüssigem Stickstoff bei niedriger Temperatur an der gefriergetrockneten dünnen Knorpelgewebescheibe, um ein Mikropartikelmaterial herzustellen, wobei die durchschnittliche Partikelgröße des Mikropartikelmaterials, bezogen auf die Anzahl der Partikel, 2 bis 20 µm beträgt und die durchschnittliche Partikelgröße des Mikropartikelmaterials, bezogen auf das Volumen der Partikel, 20 bis 200 µm beträgt;
(4) Entfetten des Mikropartikelmaterials durch Einweichen in Isopropylalkohol gemäß einem Material-Flüssigkeits-Verhältnis von 1:5 bis 1:20, Zentrifugieren und Entsorgen einer Abfallflüssigkeit, um einen ersten Niederschlag zu erhalten;
(5) Zugabe von physiologischer Kochsalzlösung oder Phosphatpuffer zu dem ersten Niederschlag in einem Material-Flüssigkeits-Verhältnis von 1:5 bis 1:20, Einweichen, Zentrifugieren und Verwerfen der Abfallflüssigkeit, um einen zweiten Niederschlag zu erhalten;
(6) Zugabe einer Desoxyribonuklease in einer Pufferlösung zu dem zweiten Niederschlag gemäß einem Material-Flüssigkeits-Verhältnis von 1:5 bis 1:20, Einweichen, Zentrifugieren und Verwerfen einer Abfallflüssigkeit, um einen dritten Niederschlag zu erhalten;
(7) Zugabe einer alkalischen Enzymlösung (Alcalase) zu dem dritten Niederschlag gemäß einem Material-Flüssigkeits-Verhältnis von 1:5 bis 1:10, Einweichen, Zentrifugieren und Verwerfen einer Abfallflüssigkeit, um einen vierten Niederschlag zu erhalten;
(8) Zugabe eines Reinigungsmittels, welches in einem Phosphatpuffer zu dem vierten Niederschlag gemäß einem Material-Flüssigkeits-Verhältnis von 1:5 bis 1:20 zusammengesetzt ist, Einweichen, Zentrifugieren und Entsorgen einer Abfallflüssigkeit, um einen fünften Niederschlag zu erhalten;
(9) Zugabe einer Dinatriumethylendiamintetraacetat-Lösung zu dem fünften Niederschlag gemäß einem Material-Flüssigkeits-Verhältnis von 1:5 bis 1:20, Einweichen, Waschen, Zentrifugieren und Entsorgen einer Abfallflüssigkeit; erneute Zugabe einer Dinatriumethylendiamintetraacetat-Lösung zum erneuten Waschen, erneutes Zentrifugieren und Entsorgen einer Abfallflüssigkeit, um einen sechsten Niederschlag zu erhalten;
(10) Zugabe von physiologischer Kochsalzlösung oder Phosphatpuffer zu dem sechsten Niederschlag in einem Material-Flüssigkeits-Verhältnis von 1:5 bis 1:20, Einweichen, Zentrifugieren und Entsorgen der Abfallflüssigkeit; erneute Zugabe von physiologischer Kochsalzlösung oder Phosphatpuffer und wiederholte Durchführung, um einen siebten Niederschlag zu erhalten;
(11) Zugabe einer gemischten Lösung aus Natriumphosphat und Peressigsäure zu dem siebten Niederschlag gemäß einem Material-Flüssigkeits-Verhältnis von 1:5 bis 1:10, um eine Virusinaktivierungsbehandlung durchzuführen, Zentrifugieren und Entsorgen einer Abfallflüssigkeit, um einen achten Niederschlag zu erhalten;
(12) Zugabe von physiologischer Kochsalzlösung oder Phosphatpuffer zum achten Präzipitat in einem Material-Flüssigkeits-Verhältnis von 1:5 bis 1:20, Einweichen, Zentrifugieren und Entsorgen der Abfallflüssigkeit, um ein neuntes Präzipitat zu erhalten; und
(13) Zugabe von sterilem Wasser zu dem neunten Niederschlag gemäß einem Material-Flüssigkeits-Verhältnis von 1:5 bis 1:20, Waschen, Gefriertrocknen und Sterilisieren mit Gammastrahlen oder Elektronenstrahlen nach dem Gefriertrocknen.

2. Verfahren zur Herstellung eines Stromamaterials zum Einkapseln von Zellen gemäß Anspruch 1, wobei: in Schritt (1) das Säugetier aus einem oder mehreren der folgenden ausgewählt ist: Schweine, Kühe, Schafe, Pferde und Hirsche;
in Schritt (1) das Knorpelmaterial aus einem oder mehreren der folgenden ausgewählt ist: elastischer Knorpel und hyaliner Knorpel;
der elastische Knorpel Ohrknorpel ist;
der hyaline Knorpel aus einem oder mehreren der folgenden ausgewählt ist: Gelenkknorpel, Rippenknorpel, Schulterblattknorpel und Meniskus.

3. Verfahren zur Herstellung eines Stromamaterials zum Einkapseln von Zellen gemäß Anspruch 1, wobei: in Schritt (2) das Desinfektionsmittel aus einem oder mehreren der folgenden ausgewählt ist: 0,1-0,5 % w/v Natriumhypochlorit, 0,5-2,0 % w/v Natriumcarbonat und 50-70 % w/v Alkohollösung;
die Alkohollösung aus einem oder mehreren der folgenden ausgewählt ist: Ethanol und Isopropanol.

4. Verfahren zur Herstellung eines Stromamaterials zum Einkapseln von Zellen gemäß Anspruch 1, wobei: in Schritt (4) das Isopropanol einen Gehalt von 50 bis 70 % (Gew./Vol.) aufweist; das Einweichen 30 bis 60 Minuten dauert und das Zentrifugieren mit einer Geschwindigkeit von 500 bis 1500 × g und für eine Zeit von 5 bis 20 Minuten erfolgt;
in Schritt (5) das Einweichen 30 bis 60 Minuten dauert und das Zentrifugieren mit einer Geschwindigkeit von 500 bis 1500 × g und für eine Zeit von 5 bis 20 Minuten erfolgt;
in Schritt (6) wird die Desoxyribonuklease-Lösung in einem Puffer zusammengesetzt, der aus einem oder mehreren der folgenden Puffer ausgewählt ist:
Hydroxyethylpiperazin-Ethansulfonsäure-Puffer und Trihydroxymethylaminomethan-Hydrochlorid-Puffer; die Desoxyribonuklease-Lösung hat eine Desoxyribonuklease-Aktivität von 50 bis 250 U/L;
der Hydroxyethylpiperazin-Ethansulfonsäure-Puffer hat eine Konzentration von 5 bis 100 mmol/l;
in Schritt (6) beträgt die Einweichzeit 8 bis 12 Stunden und die Zentrifugationsgeschwindigkeit 500 bis 1500 × g bei einer Dauer von 5 bis 20 Minuten.

5. Verfahren zur Herstellung eines Stromamaterials zum Einkapseln von Zellen gemäß Anspruch 1, wobei: in Schritt (7) die alkalische Enzymlösung (Alcalase) eine Konzentration von 0,02 bis 0,2 % w/v alkalischem Enzym (Alcalase) aufweist;
in Schritt (7) das Einweichen 30 bis 120 Minuten dauert und das Zentrifugieren mit einer Geschwindigkeit von 500 bis 1500 × g und einer Dauer von 5 bis 20 Minuten erfolgt;
in Schritt (8) das Detergens aus einem oder mehreren der folgenden ausgewählt ist: Natriumdesoxycholat, Triton X-100 und Natriumdodecylsulfat;
in Schritt (8) beträgt die Einweichzeit 8 bis 16 Stunden und die Zentrifugationsgeschwindigkeit 500 bis 1500 × g bei einer Dauer von 5 bis 20 Minuten.

6. Verfahren zur Herstellung eines Stromamaterials zum Einkapseln von Zellen gemäß Anspruch 1, wobei: in Schritt (9) die Dinatriumethylendiamintetraacetatlösung eine Konzentration von 5 bis 50 mmol/l Dinatriumethylendiamintetraacetat aufweist und in 10 mmol/l Natriumphosphatpuffer zusammengesetzt ist;
in Schritt (9) das Waschen 2-4 Stunden dauert und das Zentrifugieren mit einer Geschwindigkeit von 500-1500 × g und für eine Zeit von 5-20 Minuten erfolgt; in Schritt (9) dauert das erneute Waschen 12 bis 24 Stunden und das erneute Zentrifugieren erfolgt mit einer Geschwindigkeit von 500 bis 1500 × g und dauert 5 bis 20 Minuten;
in Schritt (10) das Einweichen 30 bis 120 Minuten dauert und die Zentrifugation mit einer Geschwindigkeit von 500 bis 1500 × g und einer Dauer von 5 bis 20 Minuten erfolgt.

7. Verfahren zur Herstellung eines Stromamaterials zum Einkapseln von Zellen gemäß Anspruch 1, wobei: in Schritt (11) die Virusinaktivierungsbehandlung 30 bis 60 Minuten dauert und die Zentrifugation mit einer Geschwindigkeit von 500 bis 1500 × g und für eine Dauer von 5 bis 20 Minuten erfolgt;
in Schritt (12) das Einweichen 30 bis 120 Minuten dauert und die Zentrifugation mit einer Geschwindigkeit von 500 bis 1500 × g und für eine Zeit von 5 bis 20 Minuten erfolgt.

8. Verfahren zur Herstellung eines Stromamaterials zum Einkapseln von Zellen gemäß einem der Ansprüche 1 bis 7, wobei: in Schritt (5), Schritt (8), Schritt (10) und Schritt (12) der Phosphatpuffer ein Natriumphosphatpuffer ist.

9. Stromamaterial zum Einkapseln von Zellen, das durch das Herstellungsverfahren gemäß einem der Ansprüche 1 bis 8 erhalten wird; Hauptkomponenten des Stromamaterials zum Einkapseln von Zellen sind Typ-II-Kollagen und Glykosaminoglykan;
das Glykosaminoglykan umfasst Chondroitinsulfat und Hyaluronsäure.

10. Stromales Material zum Einkapseln von Zellen gemäß Anspruch 9, umfassend Typ-II-Kollagen, Chondroitinsulfat und Hyaluronsäure, wobei die Summe aus Typ-II-Kollagen, Chondroitinsulfat und Hyaluronsäure über 85 Gew.-% des Trockengewichts, vorzugsweise über 90 Gew.-% des Trockengewichts des stromalen Materials zum Einkapseln von Zellen beträgt;
das Typ-II-Kollagen über 75 Gew.-% beträgt, Elastin weniger als 10 Gew.-% beträgt und das Glykosaminoglykan einen Gehalt von 1 bis 10 Gew.-% aufweist; und das hierin beschriebene Glykosaminoglykan Chondroitinsulfat und Hyaluronsäure umfasst;
das Kollagen II, das Chondroitinsulfat und die Hyaluronsäure einen Gehalt von über 92 % w/w (Trockengewicht) bzw. über 2 % w/w (Trockengewicht) bzw. über 1 % w/w (Trockengewicht) aufweisen;
die Rest-DNA im Stromamaterial zum Einkapseln von Zellen liegt bei einem Gehalt von weniger als 20 ng/mg, vorzugsweise nicht mehr als 5 ng/mg;
die Anzahl der α-Gal-Antigen-Epitope im Stromamaterial zur zum Einkapseln von Zellen liegt unter 5,0 × 10¹² /g Trockengewicht.

11. Verwendung des Stromamaterials zum Einkapseln von Zellen nach Anspruch 9 oder 10 beim 3D-Bioprinting.

12. Verwendung des Stromamaterials zur zum Einkapseln von Zellen nach Anspruch 9 oder 10 zum Einkapseln oder Schützen oder Einkapseln und Schützen von Zellen.

## Revendications

1. Procédé de préparation d'un matériau stromal destiné à encapsuler des cellules, comprenant les étapes suivantes :
(1) retirer les tissus et les fascias de la surface d'un matériau cartilagineux ex vivo provenant d'un mammifère, puis découper le matériau cartilagineux en fines tranches afin d'obtenir une fine tranche de tissu cartilagineux ;
(2) désinfecter la surface de la fine tranche de tissu cartilagineux avec un désinfectant, puis la laver et la lyophiliser ;
(3) effectuer un broyage par micronisation à basse température à l'azote liquide sur la fine tranche de tissu cartilagineux lyophilisé afin de préparer un matériau à microparticules, dans lequel la taille moyenne des particules du matériau à microparticules, basée sur le nombre de particules, est de 2 à 20 µm, et la taille moyenne des particules du matériau à microparticules, basée sur le volume des particules, est de 20 à 200 µm ;
(4) dégraisser le matériau microparticulaire en le trempant dans de l'alcool iso-propylique selon un rapport matériau-liquide de 1:5 à 1:20, centrifuger et jeter le liquide usé pour obtenir un premier précipité ;
(5) ajouter une solution saline physiologique ou un tampon phosphate au premier précipité selon un rapport matière-liquide de 1:5 à 1:20, traiter par trempage, centrifuger et jeter le liquide résiduel pour obtenir un deuxième précipité ;
(6) ajouter une désoxyribonucléase dans une solution tampon au deuxième précipité selon un rapport matière-liquide de 1:5 à 1:20, traiter par trempage, centrifuger et jeter le liquide résiduel pour obtenir un troisième précipité ;
(7) ajouter une solution d'enzyme alcaline (alcalase) au troisième précipité selon un rapport matière-liquide de 1:5 à 1:10, traiter par trempage, centrifuger et jeter le liquide résiduaire pour obtenir un quatrième précipité ;
(8) ajouter un détergent formulé dans un tampon phosphate au quatrième précipité selon un rapport matière-liquide de 1:5 à 1:20, traiter par trempage, centrifuger et jeter le liquide résiduaire pour obtenir un cinquième précipité ;
(9) ajouter une solution d'éthylènediamine tétraacétate disodique au cinquième précipité selon un rapport matière-liquide de 1:5 à 1:20, tremper, laver, centrifuger et jeter le liquide résiduaire ; ajouter à nouveau une solution d'éthy-lènediamine tétraacétate disodique pour relaver, recentrifuger et jeter le liquide résiduaire afin d'obtenir un sixième précipité ;
(10) ajouter une solution saline physiologique ou un tampon phosphate au sixième précipité selon un rapport matière-liquide de 1:5 à 1:20, laisser tremper, centrifuger et jeter le liquide résiduel ; ajouter à nouveau une solution saline physiologique ou un tampon phosphate et répéter l'opération pour obtenir un septième précipité ;
(11) ajouter une solution mixte de phosphate de sodium et d'acide peracétique au septième précipité selon un rapport matière-liquide de 1:5 à 1:10 pour effectuer un traitement d'inactivation virale, centrifuger et jeter le liquide résiduel pour obtenir un huitième précipité ;
(12) ajouter une solution saline physiologique ou un tampon phosphate au huitième précipité selon un rapport matière-liquide de 1:5 à 1:20, traiter par trempage, centrifuger et jeter le liquide résiduaire pour obtenir un neuvième précipité ; et
(13) ajouter de l'eau stérile au neuvième précipité selon un rapport matière-liquide de 1:5 à 1:20, laver, lyophiliser et stériliser aux rayons gamma ou par faisceau d'électrons après lyophilisation.

2. Procédé de préparation d'un matériau stromal pour encapsuler des cellules selon la revendication 1, dans lequel : à l'étape (1), le mammifère est choisi parmi un ou plusieurs des porcs, vaches, moutons, chevaux et cerfs ;
à l'étape (1), le matériau cartilagineux est choisi parmi un ou plusieurs des cartilages élastiques et cartilages hyalins ;
le cartilage élastique est du cartilage auriculaire ;
le cartilage hyalin est choisi parmi un ou plusieurs des cartilages articulaires, cartilages costaux, cartilages scapulaires et ménisques.

3. Procédé de préparation d'un matériau stromal pour encapsuler des cellules selon la revendication 1, dans lequel : à l'étape (2), le désinfectant est choisi parmi un ou plusieurs des composés suivants : hypochlorite de sodium à 0,1-0,5 % p/v, carbonate de sodium à 0,5-2,0 % p/v et solution d'alcool à 50-70 % p/v ;
la solution alcoolique est choisie parmi une ou plusieurs solutions parmi l'éthanol et l'isopropanol.

4. Procédé de préparation d'un matériau stromal pour encapsuler des cellules selon la revendication 1, dans lequel : à l'étape (4), l'isopropanol a une teneur de 50 à 70 % p/v ; le trempage dure 30 à 60 minutes et la centrifugation est effectuée à une vitesse de 500 à 1500 × g pendant 5 à 20 minutes ;
à l'étape (5), le trempage dure 30 à 60 minutes et la centrifugation est effectuée à une vitesse de 500 à 1500 × g pendant 5 à 20 minutes ;
à l'étape (6), la solution de désoxyribonucléase est formulée dans un tampon choisi parmi un ou plusieurs tampons à base d'hydroxyéthylpipérazine éthanesulfonique et de chlorhydrate de trihydroxyméthylaminométhane ; la solution de désoxyribonucléase a une activité de désoxyribonucléase de 50 à 250 U/L ;
le tampon d'acide hydroxyéthylpipérazineéthanesulfonique a une concentration de 5 à 100 mmol/L ;
à l'étape (6), le trempage dure entre 8 et 12 heures, et la centrifugation est effectuée à une vitesse comprise entre 500 et 1500 × g, pendant une durée comprise entre 5 et 20 minutes.

5. Procédé de préparation d'un matériau stromal pour encapsuler des cellules selon la revendication 1, dans lequel : à l'étape (7), la solution d'enzyme alcaline (alcalase) a une concentration en enzyme alcaline (alcalase) de 0,02 à 0,2 % p/v ;
à l'étape (7), le trempage dure entre 30 et 120 minutes, et la centrifugation s'effectue à une vitesse comprise entre 500 et 1500 × g, pendant une durée comprise entre 5 et 20 minutes ;
à l'étape (8), le détergent est choisi parmi un ou plusieurs des composés suivants : désoxycholate de sodium, Triton X-100 et dodécylsulfate de sodium ; à l'étape (8), le trempage dure entre 8 et 16 heures, et la centrifugation s'effectue à une vitesse comprise entre 500 et 1 500 × g, pendant une durée comprise entre 5 et 20 minutes.

6. Procédé de préparation d'un matériau stromal pour encapsuler des cellules selon la revendication 1, dans lequel : à l'étape (9), la solution d'éthylène-diaminetétraacétate disodique a une concentration en éthylènediaminetétraacétate disodique de 5 à 50 mmol/L et est formulée dans un tampon phosphate de sodium à 10 mmol/L ;
à l'étape (9), le lavage est effectué pendant une durée de 2 à 4 heures, et la centrifugation est effectuée à une vitesse de 500 à 1500 × g, pendant une durée de 5 à 20 minutes ;
à l'étape (9), le nouveau lavage dure entre 12 et 24 heures, et la nouvelle centrifugation s'effectue à une vitesse de 500 à 1500 × g pendant 5 à 20 minutes ;
à l'étape (10), le trempage dure entre 30 et 120 minutes, et la centrifugation s'effectue à une vitesse comprise entre 500 et 1500 × g, pendant une durée comprise entre 5 et 20 minutes.

7. Procédé de préparation d'un matériau stromal pour encapsuler des cellules selon la revendication 1, dans lequel : à l'étape (11), le traitement d'inactivation virale dure entre 30 et 60 minutes, et la centrifugation s'effectue à une vitesse comprise entre 500 et 1500 × g pendant une durée comprise entre 5 et 20 minutes ;
à l'étape (12), le trempage dure entre 30 et 120 minutes, et la centrifugation s'effectue à une vitesse comprise entre 500 et 1500 × g pendant une durée comprise entre 5 et 20 minutes.

8. Procédé de préparation d'un matériau stromal pour encapsuler des cellules selon l'une quelconque des revendications 1 à 7, dans lequel : dans les étapes (5), (8), (10) et (12), le tampon phosphate est un tampon phosphate de sodium.

9. Matériau stromal pour encapsuler des cellules obtenu par le procédé de préparation selon l'une quelconque des revendications 1 à 8 ;
les principaux composants du matériau stromal pour encapsuler des cellules sont le collagène de type II et le glycosaminoglycane ;
le glycosaminoglycane comprend du sulfate de chondroïtine et de l'acide hyaluronique.

10. Matériau stromal pour encapsuler des cellules selon la revendication 9, comprenant du collagène de type II, du sulfate de chondroïtine et de l'acide hyaluronique, dans lequel la somme du collagène de type II, du sulfate de chondroïtine et de l'acide hyaluronique est supérieure à 85 % en poids/poids sec, de préférence supérieure à 90 % en poids/poids sec du matériau stromal pour encapsuler des cellules ;
le collagène de type II est supérieur à 75 % p/p, l'élastine est inférieure à 10 % p/p et le glycosaminoglycane a une teneur comprise entre 1 % et 10 % p/p ; et le glycosaminoglycane décrit ici comprend du sulfate de chondroïtine et de l'acide hyaluronique ;
le collagène II, le sulfate de chondroïtine et l'acide hyaluronique ont une teneur supérieure à 92 % p/p ( ) (poids sec), supérieure à 2 % p/p (poids sec) et supérieure à 1 % p/p (poids sec), respectivement ;
l'ADN résiduel dans le matériau stromal destiné à encapsuler les cellules est présent à une teneur inférieure à 20 ng/mg, de préférence inférieure à 5 ng/mg ;
les épitopes de l'antigène α-Gal dans le matériau stromal destiné à encapsuler les cellules sont en quantité inférieure à 5,0×10¹² /g de poids sec.

11. Utilisation du matériau stromal pour encapsuler des cellules selon la revendication 9 ou 10 dans l'impression biologique 3D.

12. Utilisation du matériau stromal pour encapsuler des cellules selon la revendication 9 ou 10 dans l'encapsulation, la protection ou l'encapsulation et la protection de cellules.
